# EUROPEAN PATENT SPECIFICATION

(11) **EP 1 978 917 B1**
(45) Date of publication and mention of the grant of the patent: **19.10.2011**
(21) Application number: 06829908.0
(22) Date of filing: 27.12.2006
(51) Int. Cl.: A61K 8/06, A61K 8/26, A61K 8/33, A61K 8/34, A61K 8/36, A61Q 15/00

(54) **ANTIPERSPIRANT COMPOSITIONS**
ANTIPERSPIRANS-ZUSAMMENSETZUNGEN
COMPOSITIONS ANTI-TRANSPIRANTES

(30) Priority: 27.01.2006 GB 0601644
(43) Date of publication of application: 15.10.2008
(73) Proprietor: Unilever PLC, London EC4Y 0DY (GB); Unilever N.V., 3013 AL Rotterdam (NL)
(72) Inventor: FRANKLIN, Kevin, Ronald, Bebington, Wirral Merseyside CH63 3JW (GB); IRVOAS, Anne-Cecile, Marie, Eugenie, Bebington, Wirral Merseyside CH63 3JW (GB)
(74) Representative: Whaley, Christopher
(86) International application number: PCT/EP2006/012651
(87) International publication number: WO 2007/085299

(56) References cited:
- WO-A-2005/063188
- US-A1- 2003 053 970
- US-A1- 2005 152 861

## Description

The present invention relates to non-therapeutic antiperspirant compositions and more particularly to emulsion compositions.

Non-therapeutic antiperspirant compositions are applied topically to the skin in regions of the body where the appearance of sweat is considered to be unsightly. The most common region is the axilla, otherwise called the armpit. Such compositions can adopt various forms depending on their manner of manufacture and the type of dispenser from which they are applied. Three principal forms comprise sticks or creams, powders and liquids.

The instant invention relates particularly to liquid compositions. These can be applied from contact dispensers, of which the most widely available dispenser is often called a roll-on, or from an impregnated sheet or as a spray, the latter being created by propelling a liquid stream of the composition through a narrow orifice that transforms the stream into droplets. The stream can be propelled by mechanical force, as in a pump spray or squeeze spray or by pressure generated within the dispenser by a propellant.

Liquid antiperspirant compositions can comprise a suspension of a particulate antiperspirant active in a carrier liquid in which it is not soluble, a solution of the antiperspirant active in a hydrophilic liquid, often comprising ethanol, or an emulsion in which the antiperspirant active is soluble in one of the phases, commonly an aqueous phase. Each class of liquid compositions has its own combinations of benefits and disadvantages, including constraints on additional ingredients which can be incorporated into the compositions to render the compositions more desirable to consumers. Antiperspirant solutions tend to be rather acidic by virtue of the nature of water-soluble materials that provide effective antiperspirancy, which constrains the materials from which dispensers can be made, and especially pressurised containers, and additionally their application can be perceived as wet with poor drying properties. Suspensions of particulate materials can suffer from agglomeration, introducing a gritty feel. Emulsions can suffer from a combination of problems; the acidity and wetness of an aqueous phase and oiliness or greasiness arising from an oil phase.

The market for antiperspirant compositions is not unitary, but can be segmented, inter alia, into products that promote optimum antiperspirant efficacy, those which avoid or minimise an unsightly appearance on application (reduced visible deposits) and those which offer improved sensory benefits. Some products are alleged to combine optimum efficacy with reduced visible deposits.

Emulsions contemplated herein comprise two distinct phases, a continuous phase in which droplets of a second phase is suspended. The sensory properties of the resultant emulsion differ depending on which constitutes the external (otherwise called continuous) phase. The sub-class of oil in water emulsions are especially prone to feel wet, and to dry slowly when topically applied, and the range of materials for making dispensers for such compositions is curtailed by the potentially corrosive nature of the external phase. On the other hand, if the emulsion is not properly balanced, water in oil emulsions, the subclass contemplated herein, can suffer from oily, greasy, draggy, heavy or sticky sensations on topical application. The inventive skill of the inventor commonly resides in how he selects and combines the composition ingredients to maintain their efficacy whilst improving the overall balance of sensory properties.

It has been disclosed that visible deposits can be reduced by incorporating into antiperspirant compositions water-immiscible oils having a comparatively high refractive index, but such oils have side-effects, such as increasingly the likelihood that the resultant compositions will feel sticky and possibly greasy and/or oily, though the latter attribute is not always viewed negatively.

The present invention relates to compositions which offer sensory benefits and in particular to those which contain a preferred class of skin moisturiser, namely a humectant, and particularly a polyhydric humectant, i.e. containing at least 2 hydroxyl substituents. It is desirable to select the contents of humectant and the antiperspirant active in proportion to each other, because, to a least some extent, the humectant is intended to counteract demoisturising effect of the active.

The present invention is seeking to ameliorate or overcome sensory disadvantages arising from employing an emulsion of a water-soluble antiperspirant active and a polyhydric humectant.

### Brief summary of the present invention

According to the present invention, there is provided an aerosol composition comprising a liquefiable propellant and a base composition characterised the liquefiable propellant is present in a proportion of 40 to 88% by weight of the aerosol composition and the base composition is in the form of a liquid emulsion comprising a continuous oil phase, a dispersed aqueous phase and an emulsifier

The base composition is suitably characterised by one or more of the following:-
said aqueous phase represents from 45 to 80% by weight of said aqueous phase
said aqueous phase comprises from 20 to 50% of a water-soluble astringent antiperspirant active salt, based on the base composition
said aqueous phase comprises from 2 to 20% of a polyhydric humectant, based on the base composition;
said oil phase represents from 20 to 55% of the base composition
said oil phase comprises a volatile silicone oil in an proportion of at least 25% of the oil phase;
said oil phase comprises a sensory modifying oil selected from dialkyl ethers and dialkyl carbonates having a boiling point of at least 250°C in an proportion of at least 20% of the oil phase, preferably in a weight ratio to the polyhydric humectant of from 3:4 to 4:1, said emulsifier is a silicone polyol preferably present in an amount of at least 0.15% of the base composition.

Herein, all percentages in the text are by weight, unless expressly stated otherwise. The weight percentages are based on the base composition except where stated otherwise. Some percentages are expressly based on the individual phase within the base composition.

By the appropriate selection of the proportions of the phases, the content of particular ingredients within such phases at selected concentrations and ratios, it is possible to achieve a balance of sensory properties to at least some extent, thereby at least ameliorating the sensory properties of antiperspirant emulsions containing a moisturiser.

### Detailed Description of the Inventions and Preferred Embodiments thereof.

The present invention relates to liquid water in oil emulsions containing an antiperspirant astringent salt, a polyhydric humectant, and an oil phase comprising both a volatile silicone and a sensory modifying oil. It is particularly desirable for the polyhydric humectant to be present in a weight ratio to the antiperspirant salt that meets or exceeds a threshold ratio and especially suitably, the contents of the polyhydric humectant and the sensory modifying oil are selected within a defined range of ratios so as to achieve a desirable balance of sensory properties. Such compositions represent base compositions which can be mixed with liquifiable propellants in a particularly desirable aspect of the invention to form aerosol compositions.

### Base Composition

Herein, the base composition comprises three constituent parts, namely an oil phase, an aqueous phase and an emulsifier that is located at the interface between the oil and aqueous phases.

### Oil Phase

The oil phase herein normally represents from 20 to 55% by weight, oil phase %s herein being by weight of the base composition. In many desirable compositions, the oil phase represents at least 30%, particularly greater than 30%. Often in such or other desirable compositions, the oil phase represents no more than 50%, and particularly less than 50%. In a number of embodiments demonstrating an advantageous combination of sensory properties, the oil phase represents no more than or particularly less than 40%. Especially desirably the oil phase represents at least 32%. Within any class of oils, a single oil or a mixture of 2 or more oils can be contemplated by the producer of the base composition. Herein, the oil phase comprises a mixture of hydrophobic, water-immiscible oils. Such oils include a volatile oil, and a sensory modifying oil, and can additionally comprise one or more of a non-volatile oil, especially one having visible residue masking properties, and water-immiscible components of a fragrance. By volatile herein is meant having a measurable vapour pressure at 20 or 25°C. It is particularly preferred to select the components of the oil phase and their content therein to balance the sensory properties of the resultant composition. Herein, %s of such oils are by weight based on the oil phase, unless otherwise expressly stated.

The volatile oil is especially desirably a volatile silicone oil. Typically the vapour pressure of a volatile silicone oil lies in a range from 1 or 10 Pa to 2 kPa at 25°C. Volatile silicone oils can be linear or cyclic siloxanes, usually containing from 3 to 9 silicon atoms, and commonly from 4 to 6 silicon atoms, the silicon atoms being substituted by methyl groups, so that their alternative names are methicones and cyclomethicones. It is especially desirable to employ volatile silicone oils in which at least 80% by weight thereof and particularly at least 90% by weight thereof contain at least 5 silicon atoms, such as cyclopentadimethylsiloxane (D5), cyclohexadimethylsiloxane (D6), dodecamethylpentasiloxane and tetradecamethylhexasiloxane. The cylomethicone oils are especially preferred. Such oils are highly desirable for many consumers because they can evaporate without causing undue skin cooling.

The volatile silicone oil advantageously represents at least, and particularly greater than, 25% of the oil phase, and in many highly desirable embodiments represents no more than, or particularly less than, 70%. In some preferred embodiments, the volatile oil constitutes at least, or particularly greater than, 30%.

A second component of the oil phase is the sensory modifying oil. In conjunction with the volatile oil, the presence of the sensory modifying oil modifies the perception of the antiperspirant composition when it is topically applied to human skin. The identification of suitable oils for this purpose and in particular the selection of dicaprylyl ether and dicaprylyl carbonate is not something that the skilled man could derive from the vast wealth of prior disclosures of antiperspirant formulations without the exercise of inventive effort. Many and varied are the classes of non-volatile oils that have been disclosed for incorporation in antiperspirant compositions, including emulsions, their virtues being extolled. Thus, for example, some of such classes or at least representative oils within them are asserted to demonstrate properties of reducing the appearance of visible residues. Other oils are asserted to have a different sensation of oiliness or greasiness or to affect the stability of the compositions. However, none of the formulations comprise the combination of emulsion components identified in the instant invention and none direct the skilled man to the beneficial combination of sensory properties enjoyed by such compositions.

The sensory modifying oils employable herein have a high boiling point, herein at 1 atmosphere pressure, commonly up to 420°C. This imposes constraints on preferred alkyl substituents of such oils. Desirably such substituents are linear. Contemplatable substituents include octyl and decyl. Particularly preferred sensory modifying oils include dioctyl ether and dioctyl carbonate, which preferably constitute at least half of the sensory modifying oils, and particularly at least four fifths, by weight. These two compounds are also known as dicaprylyl ether and dicaprylyl carbonate. In some highly desirable embodiments, the sensory modifying oil or mixture of oils has a boiling point of at least 280°C, and conveniently in the range of from 280 to 370°C.

The sensory modifying oils advantageously comprise at least or particularly greater than 20% of the oil phase, preferably up to 35%, and especially below 35%. It is beneficial in desirable embodiments to select the proportion of the sensory modifying oil or mixture of oils in relation to the amount of polyhydric humectant that is present in the composition. Preferably, in at least some desirable embodiments, there are at least 3 parts by weight of the sensory modifying oil per 4 parts by weight of the said humectant. In the same or other desirable embodiments, the weight ratio is up to 4 parts of sensory modifying oil per part of said humectant, by weight. The sensory modifier often is present in excess weight, compared with the humectant. The weight ratio of the modifier to the humectant is particularly at least 6:5 and is especially preferably up to 2:1.

An optional component of the oil phase comprises an ester oil, and preferably an ester oil which is selected from triglyceride ester oils and ester oils that have a refractive index at 25°C of at least 1.48. Triglyceride ester oils preferably derive from aliphatic acids containing at least 6 carbon atoms.

It is particularly suitable to employ triglyceride oil derived from unsaturated fatty acids, and in particular from such acids that contain 18 carbon atoms, including various of such ester oils commonly available as extractions from plants. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The presence of the triglyceride ester oils and particularly the oils derived from unsaturated acids improves the ability of the skin to ameliorate the effects of hair removal, such as plucking or shaving, for example in the underarm, for example by at least partly avoiding irritation or assisting the skin to recover from being irritated.

The oil phase can additionally comprise one or more fragrance oils, for example in an amount of up to 4% by weight of the base composition.

The oil phase can additionally comprise a minor fraction of an aerosol spray modifier, which, if desired, can be a very high molecular weight silicone often in the physical form of a gum. The amount of such spray modifier, if present, is commonly selected in the range of from 0.2 to 1.5% by weight of the oil phase.

### Aqueous Phase.

In the invention base compositions, the aqueous phase represents the dispersed phase. Commonly herein, this phase constitutes at least 45%, and desirably not more than 80%, %s being by weight of the base composition. (Herein, the term base composition may be abbreviated to base.) In many suitable embodiments, the aqueous phase constitutes greater than 50% by weight of the base, and in such or other desirable embodiments, the aqueous phase constitutes less than 70% of the base.

As will be apparent hereinafter, it is particularly suitable to select the proportion of aqueous phase in the aerosol composition in conjunction with the proportion of propellant in that aerosol composition. Thus, aerosol compositions employing a high proportion of propellant preferably employ base compositions having a comparatively high proportion of aqueous phase, aerosol compositions with an intermediate proportion of propellant conveniently employ a base with an intermediate proportion of aqueous phase and aerosol compositions with a low proportion of propellant can conveniently employ a low proportion of aqueous phase.

The antiperspirant active salt is dissolved in the aqueous phase. Its practical maximum concentration is commonly in the region of 55%, the precise ceiling depending on the nature of other constituents therein. This constrains the total proportion of antiperspirant salt that can be accommodated with an emulsion. The residue of the aqueous phase accommodates the humectant, the sensory properties of the composition becoming impaired as the concentration of humectant in the aqueous phase increases.

Without being bound to any particular theory, preferences for proportions of the phases mentioned herein take into account the perceived habits of consumers and accordingly increase the likelihood that the users will topically apply an efficacious dose of the composition coupled with a desirable amount of humectant.

The antiperspirant salt can be an astringent metal salt that is water soluble. At the discretion of the producer and desirably in accordance with practices where the product is intended to be sold and/or employed, the astringent salt is preferably selected from astringent salts of aluminium and/or zirconium, or if desired, water-soluble astringent titanium salts can be contemplated as an alternative. In some embodiments, the salt is an aluminium-zirconium salt and in other very desirable compositions, especially when incorporated in aerosol compositions, the astringent salt is an aluminium salt. It is especially desirable that the astringent metal salt is basic, by which is meant that a fraction of the counterion is hydroxyl, preferably at least 30 molar % of the counterion particularly up to 92 molar % and in many valuable embodiments from 65 to 85 molar %.

The counterion in many desirable antiperspirant salts comprises a halogen, such as chloride or bromide, and especially comprises chloride.

In a number of preferred compositions, it is especially desirable to employ an aluminium chlorohydrate, by which is meant herein a material which satisfies the empirical formula Al₂(OH)ₓCl_{y} in which x + y = 6 and y is normally at least 0.5 and commonly not greater than 1.8, the material usually comprising bound water of hydration. The weight proportion of said water of hydration is conventionally not more than 12% and often lies in the range of from 3 to 10%. The term aluminium chlorohydrate herein encompasses materials with specified figures for x and y, such as aluminium sesquichlorohydrate and materials in which the chlorohydrate is present as a complex. It will be recognised that alternative names are sometimes used to indicate the presence of hydroxyl substitution, including aluminium hydroxychloride, aluminium oxychloride or basic aluminium chloride.

Aluminium chlorohydrate as made comprises a mixture of a number of different polymeric species in varying proportions, depending on the molar ratio of aluminium to chloride and the conditions employed during manufacture. All such mixtures are employable herein, including if desired what is commonly called activated aluminium chlorohydrate or enhanced activity aluminium chlorohydrate, sometimes abbreviated to AACH, in which the proportion of the more active species is initially higher by virtue of its method of manufacture.

Aluminium chlorohydrate contained in invention compositions can be complexed, whereupon the CTFA name for the complex is concatenated to aluminium chlorhydrex, followed by the name of the molecule with which it is complexed. Commonly, such complexes include propylene glycol, representative of C₂ to C₆ glycols and glycine, a representative of aminoacids.

In other embodiments, for example compositions intended for use in contact dispensers, the antiperspirant salt advantageously comprises a basic aluminium/zirconium salt, and particularly an aluminium/zirconium chlorohydrate. In such antiperspirant salts, the Al:Zr molar ratio is commonly selected in the range of from 2:1 to 10:1 and especially up to 6:1. The counterion content, and especially chloride, in many desirable Al/Zr antiperspirant salts can be expressed as an Al:Cl ratio of from 2.1:1 to 0.9:1. In various highly desirable compositions, the metal (Al + Zr) mole ratio to chloride is from 1.3:1 up to 1.5:1. In some others, a lower mole ratio can be contemplated, such as from 0.9:1 to <1.3:1. Particularly efficacious A/Zr antiperspirant salts comprise a complex with an aminoacid, and particularly with glycine.

Herein, where the antiperspirant salt comprises a complex with a further molecule, for example glycine, the weight of the complexed molecule is included in the weight of the antiperspirant salt when determining the proportion of the salt in the composition.

The aqueous phase in the base compositions herein often contains water in a weight ratio to the antiperspirant salt of from 0.9:1 to 1.1:1.

The aqueous phase contains the water-soluble humectant. Such humectant herein is a polyol, by which is meant herein an aliphatic alcohol containing at least 3 carbon atoms and at least 2 hydroxyl groups, optionally containing one or more ether linkages, and the number of oxygen atoms (Hₒ) in the humectant are at least half (H_{c} + 1) where H_{c} is the number of carbon atoms in the humectant. Desirably, the molecular weight of the humectant is from 76 to 450, or even up to 600. Suitable humectants herein include propylene glycol -and sorbitol, and a particularly preferred humectant is glycerol. A further advantageous class of humectants is polyethylene glycol, particularly polymers that are liquid at 25°C, and advantageously members of the class containing on average from 4 to 12 glycol mers, such as PEG-4, PEG-6, or PEG-8. Mixtures of said humectants can be employed, if desired, including for example a mixture of glycerol and a polyethylene glycol.

The proportion of humectant in the base composition is normally from 2 to 20% by weight. In a number of desirable embodiments, it is less than 15%, and a particularly convenient proportion is at least 4%, and particularly greater than 4%. In certain desirable embodiments, it is up to 7.5% or notably less than 7.5%. Often the weight proportion of the humectant is at least or notably greater than 5% up to 12% or particularly less than 12% of the base composition. Selection within the range of >5% to <12% enables substantial formulation space for other constituents and offers an excellent balance in sensory properties for the composition.

The proportion of the aqueous phase constituted by the humectant is commonly less than 30%. In many desirable embodiments, said proportion is greater than 10%.

As indicated hereinbefore, it is desirable to select the proportion of humectant and especially glycerol in conjunction with the proportion of the sensory modifying oil, even though they are in separate phases in the composition. Naturally, both the oil and the humectant are each advantageously selected within their specified ranges or preferred ranges.

The aqueous phase can be obtained by mixing an aqueous solution of the antiperspirant salt with the humectant, and optionally diluted with water. The aqueous phase can additionally comprise a water-soluble component of a fragrance. Commonly, such water-soluble fractions comprise up to 0.5% by weight of the base composition.

### Emulsifier

In the instant base compositions; a third component is an emulsifier to form a water-in-oil emulsion. Such emulsifiers are known to the skilled person to have a comparatively low HLB value, often no higher than 6.5. Whilst a single emulsifier can be employed, a mixture of emulsifiers is similarly conceivable, the weighted average HLB value for the mixture most desirably having the low HLB value. The emulsifier can if desired be selected from the gamut of classes of non-ionic emulsifiers, including polyoxyalkylene or polyol fatty ethers or esters, including in particular polyoxyethylene or polyoxypropylene alkyl ethers or esters containing a small number of such oxyalkylene units, eg 2 to 5, and an alkyl of at least 12 carbon atoms length. However, for the instant compositions which contain a volatile silicone oil, it is especially desirable to employ a silicone copolyol and especially a dimethicone copolyol or an alkylmethicone copolyol. Preferred examples of suitable silicone copolyols include emulsifiers having the trade names DC3225C and DC5225C from Dow Corning, and those under the trade names Abil EM90 and Abil EM97 from Goldschmidt (Degussa).

The proportion of emulsifier or mixture, in the composition is often selected in the range of at least 0.15%, preferably at least 0.2% and especially at least 0.3%. Whilst the upper limit for the emulsifier is at the discretion of the formulator, it is commonly less than 2% and in many convenient embodiments is less than 1%, and especially in conjunction with the selection of a silicone copolyol.

### Propellant

In the aerosol compositions herein that are made by mixing a base composition with a propellant, the proportion of propellant is normally at least 40% by weight of the resultant aerosol composition. In many compositions the propellant constitutes up to 88% of the aerosol composition. The range of aerosol compositions can be considered to fall into three camps. One camp relates to compositions with a comparatively high propellant content, a second camp to compositions containing an intermediate propellant content and a third camp relates to compositions with a low propellant content.

It is desirable to consider the relationship of the disperse aqueous to continuous oil phases and/or the proportion of volatile oil in the oil phase in relation to the proportion of propellant in the aerosol composition. In order to most easily achieve a desirable balance of sensory properties, it is particularly desirable in respect of the first camp in which the base composition represents up to 1/3^{rd} by weight of the aerosol composition for the oil and aqueous phases to be selected in a range of weight ratio of from 1:2 to 3:4. It is preferable for the volatile silicone content of the oil phase to be from 30 to 45% of that phase. In many desirable embodiments, those two factors are considered together such that the volatile silicone content of the base composition is from 11 to 15%.

In relation to the second camp of aerosol compositions, in which the base composition represents from less than 1/3^{rd} to 9/20ths of the aerosol composition, it is preferable for the oil and aqueous phases to be present in a weight ratio of from 3:5 to 1:1. In the compositions with the intermediate propellant content, the concentration of volatile silicone is very conveniently from 40 to 50% of the oil phase. In many desirable embodiments, those two factors are considered together such that the volatile silicone content of the base composition is from 16 to 23%.

In relation to the third camp of aerosol compositions with a low propellant content, in which the base composition represents from greater than 9/20ths to 3/5ths of the aerosol composition, it is preferable for the oil and aqueous phases to be present in a weight ratio of from 2:3 to 1:1. In the compositions with the intermediate propellant content, the concentration of volatile silicone is very conveniently from 45 to 65% of the oil phase. In many desirable embodiments, those two factors are considered together such that the volatile silicone content of the base composition is from 21 to 29%.

The propellant is conveniently a low boiling point material or mixture of materials, typically boiling below -5°C, and often below -15°C, and commonly above -50°C. In particular, suitable propellants comprise alkanes and/or halogenated hydrocarbons. Examples of suitable alkanes for employment, usually in varying admixture with each other include particularly propane, butane and isobutane, often in varying admixtures of the three components, possibly containing a fraction of pentane or isopentane. Examples of halogenated hydrocarbons are fluorocarbons and chlorofluorocarbons such as, for example, 1,1-difluoroethane, 1-trifluoro-2-fluoroethane, dichlorodifluoromethane, 1-chloro-1,1-difluoroethane, and 1,1-dichloro,-1,1,2,2-tetrafluoroethane.

The instant compositions can be made by methods that are conventionally known for the preparation of respectively liquid antiperspirant emulsions and aerosol compositions. Thus, the base compositions are readily made by first preparing separately an oil phase, by mixing together the miscible oils conveniently also containing the emulsifier or at least the emulsifier with the lower HLB if a mixture of emulsifiers is contemplated, and a separate aqueous phase by mixture of an aqueous solution of an antiperspirant salt with the humectant, and optionally additional aqueous diluent. The antiperspirant salt solution can be preformed. The two phases are then brought together in the presence of the emulsifier, which may have been incorporated in eg the oil phase or added separately, under conditions usually of high shear (conventionally ≥ 1500 sec⁻¹) in order to form droplets from one of the phases. In the instance of the instant invention base compositions, it is the aqueous phase that is dispersed. Fragrance is added at a time of convenience to the formulator. This may be with gentler stirring after the high shear emulsification step or even by subsequent addition into a canister that has been dosed with the residue of the base composition. The preparation of the base emulsion is carried out in the vicinity of ambient temperature, such as is commonly in the range of from 15 to 25°C.

The invention aerosol compositions are conveniently prepared by charging an aerosol canister with a base composition, fitting the canister with a valved outlet line and then charging the propellant through the outlet line, mixing it with the base composition. In order to function as an aerosol, an actuator is then fitted to the canister so that it can open the valve and permit the canister contents to be discharged.

The skilled person will also recognise that depending on the eventual viscosity of the base composition, which in turn can sometimes depend on the extent of shearing to which the base composition was subjected during its preparation, those a lower viscosity can be sprayed by from a pump or squeeze spray, whereas those base compositions of an intermediate viscosity may be suitable for dispensing through a roll-on.

The instant compositions can be applied topically to human skin as a non-therapeutic method for at least ameliorating perspiration and/or the generation of malodour compounds from skin gland secretions, especially in those areas of the body such as in the underarm where there is a high density of eccrine glands. The compositions can be applied in a conventional manner, such as directing aerosol compositions towards the selected area of the body at a distance typically of about 15 cms for a suitable discharge period, under the control of the consumer, which for many, if not most, users is from 0.5 to 10 seconds per armpit.

Hereinbefore, unless otherwise specified or clear from the context, properties are quoted at standard pressure and/or temperature and proportions, percentages and amounts quoted numerically are approximate.

Having given a summary of the invention and a detailed description of preferences, particular embodiments will hereinafter be described more fully by way of example only. Compositions according to the present invention, prefixed by Ex, and comparison compositions, prefixed by C herein were made by the following general method:-

In a first stage, base compositions were made at laboratory ambient temperature by first preparing separately an oil phase, by mixing together the miscible oils and the emulsifier and optionally any spray modifier and an aqueous phase.by mixture of an aqueous solution of an antiperspirant salt with the humectant, and optionally further water, the ingredients being employed in the weight proportions specified. The water phase was then introduced into the oil phase under conditions usually of high shear in a Silverson mixer (about 2000 sec⁻¹) in order to disperse the aqueous phase. Fragrance was then mixed in using more gentle stirring. In a second stage, the base emulsion was charged into a conventional aerosol canister, a conventional discharge line equipped with a one way valve was fitted and the canister then charged with propellant in the weight ratio of propellant to base composition specified.

In the Example and Comparison compositions herein, the ingredients are identified in Table 1 below.

**Table 1**

| **Name** | **Supplier** | **Trade Mark** |
|---|---|---|
| Cyclomethicone | Dow Corning | DC245 |
| C₁₂-₁₅ alkyl benzoate | Finetex | Finsolv TN |
| Dioctyl ether | Cognis | Cetiol OE |
| Dioctyl carbonate | Cognis | Detiol CC |
| Isopropyl Myristate | Uniqema | Estol 1514 |
| Hydrogenated Polydecene | **Amoco** | Silkflo 364NF |
| Sunflower Seed Oil | Cargill | Agri Pure 80 |
| Silicone Gum | Dow Corning | Q2-1501 |
| Glycerin | Uniqema | Pricerine 9091 |
| Polyglycol | **Clariant** | PEG400 |
| Aluminium chlorohydrate (50% aq. solution) | B G Giulini | Aloxicol L |
| Water (demineralised) | own production | |
| emulsifier | Goldschmidt | Abil EM90 |
| Fragrance | | |
| Propellant (butane, isobutane, propane) | Calor/BP | CAP 40 |

### Example 1 and Comparison C1

The compositions of these comparisons and Example are summarised in Table 2 below, together with an assessment of certain important sensory properties of those compositions in Table 3. The assessments were made by a panel of trained assessors. For each assessment, the volar forearms of a panellist were sprayed from a distance of 15 cms for 4 seconds, one with the Example and the other with a comparison. The left/right variation was balanced out over the panel. The panellists assessed the wetness, stickiness (an attribute which develops with time) and greasiness/oiliness and the head to head results are shown. +ve indicates that the Example score is higher and -ve that it is lower.

**Table 2**

| | **C 1** | **Ex 1** |
|---|---|---|
| **Ingredients** | **% w/w** | **% w/w** |
| Cyclomethicone | 13.41 | 13.41 |
| Fragrance | 0.80 | 0.80 |
| C₁₂-₁₅ alkyl benzoate | 2.00 | 2.00 |
| Dioctyl ether | | 6.67 |
| Glycerol | | 3.33 |
| ACH solution) | 20.00 | 20.00 |
| Water | 13.34 | 3.34 |
| Emulsifier | 0.25 | 0.25 |
| Silicone gum | 0.2 | 0.2 |
| Propellant | 50.00 | 50.00 |

**Table 3**

| Panel Score | Ex1 vs C 1 |
|---|---|
| Wet on application | -1.7 |
| Wet 1 min | -1.4 |
| Wet 2 min | -1.2 |
| Sticky 1 min | -0.6 |
| Sticky 2 min | -1.0 |
| Greasy / oily 1 min | +0.9 |
| Greasy / oily 2 min | +0.9 |

From Table 3 it can be seen that the incorporation of glycerol reduced the feeling of wetness compared with the comparison composition, but that at the same time stickiness, was also reduced and the sensation of greasiness/oiliness did not increase as much as might be expected. This excellent balance of properties was attributed to the incorporation of the dioctyl ether in the Example composition.

### Example 2 and Comparisons C2 to C5

The formulations for these compositions are summarised in Table 4 below.

**Table 4**

| | **C 2** | **C 3** | **C 4** | **C 5** | **Ex 2** |
|---|---|---|---|---|---|
| Ingredients | % w/w | % w/w | % w/w | % w/w | % w/w |
| cyclomethicone | 13.41 | 13.41 | 13.41 | 13.41 | 13.41 |
| Fragrance | 0.80 | 0.80 | 0.80 | 0.80 | 0.80 |
| C₁₂₋₁₅ alkyl benzoate | 2.00 | 2.00 | 2.00 | 8.67 | 2.00 |
| Dioctyl ether | 13.34 | | | | 6.67 |
| Isopropyl Myristate | | | 6.67 | | |
| Glycerol | | 13.34 | 6.67 | 6.67 | 6.67 |
| ACH solution | 20.00 | 20.00 | 20.00 | 20.00 | 20.00 |
| Emulsifier | 0.25 | 0.25 | 0.25 | 0.25 | 0.25 |
| Silicone gum | 0.2 | 0.2 | 0.2 | 0.2 | 0.2 |
| Propellant | 50.00 | 50.00 | 50.00 | 50.00 | 50.00 |

In these comparisons and Example, the compositions were tested by a small ad hoc panel of research scientists into antiperspirant formulations, not formally trained to the standard of the assessment panel participants, but experienced in differentiating between the sensory properties of antiperspirant compositions. The ad hoc panel preferred Example 2 to both comparisons C2 and C3 because C2 was judged to be very sticky and C3 to be very sticky, by comparison. Example 2 was judged to be only slightly oily and very slightly sticky after about 2 minutes. The example composition contained a total of 13.34% in total of equal amounts of dioctyl ether and glycerol, whereas the comparisons contained 13.34% of either one or the other. This comparison shows that the composition according to the invention with both ingredients provides a superior balance of properties than if either the sensory modifier or humectant is omitted.

The ad hoc panel considered that Example 2 was preferable to Comparisons C4 and C5, because they felt very heavy (greasy) upon and after application (1-2 minutes) and at peak stickiness were more sticky during dry down.

The same ad hoc panel also compared Example 2 with Example 1. The two Example compositions were felt to enjoy similar wetness (and in that attribute, Example 1 was felt to be better than the comparison C1), though Ex1 was considered to be slightly less sticky and less oily.

### Example 3

In this Example, the composition of Ex 1 was repeated, but dioctyl ether was replaced by the same weight of dioctyl carbonate. The ad hoc panel compared the two Examples and was unable to detect a difference in properties between them.

### Example 4 and Comparison C6

Compositions that employ an intermediate proportion of propellant are summarised in Table 5 below.

**Table 5**

| **Code** | **C 6** | **Ex 4** |
|---|---|---|
| **Ingredients** | **% w/w** | **% w/w** |
| Cyclomethicone | 8.41 | 5.06 |
| Fragrance | 0.80 | 0.80 |
| C₁₂-₁₅ alkyl benzoate | 2.00 | |
| Dioctyl ether | | 4.0 |
| Sunflower Seed Oil | | 3.25 |
| Glycerol | | 3.34 |
| ACH solution | 20.00 | 18.00 |
| Water | 3.34 | |
| Emulsifier | 0.25 | 0.25 |
| Silicone gum | 0.2 | 0.2 |
| Propellant | 65.00 | 65.00 |

The ad-hoc panel preferred Ex 4 to comparison C6 because it felt less wet, dried more quickly and was very smooth. Thus once again, the invention composition provided a superior balance of sensory properties.

### Example 5 and Comparisons C7 and C8

Compositions that employ a still higher proportion of propellant are summarised in Table 6 below.

**Table 6**

| | **Ex 5** | **C 7** | **C 8** |
|---|---|---|---|
| **Ingredients** | **% w/w** | **% w/w** | **% w/w** |
| Cyclomethicone | 4.06 | 4.06 | 3.41 |
| Fragrance | 0.80 | 0.8 | 0.8 |
| Dioctyl ether | 3.0 | 2.0 | 2.0 |
| Glycerol | 3.34 | 3.34 | 3.35 |
| Sunflower Seed Oil | 3.35 | 3.35 | 2.0 |
| ACH solution | 20.00 | 16.00 | 16.00 |
| Emulsifier | 0.25 | 0.25 | 0.25 |
| Silicone gum | 0.2 | 0.2 | 0.2 |
| Propellant | 69.0 | 70.0 | 72 |

The composition of Example 5 was considered by the ad-hoc panel to be superior to that of comparisons C7 and C8 because the latter underwent a very sticky maximum stickiness during dry-down, an undesirable negative attribute from which the invention composition did not suffer.

### Example 6 and Comparison C9

In this Example and comparison the sensory attributes of two compositions were assessed as described for Example 1 and Comparison C1, employing a ratio of dioctyl ether to glycerol respectively according to and not according to the instant invention. The compositions are summarised in Table 7 below and the assessment summarised in Table 8.

**Table 7**

| | **Ex 6** | **C9** |
|---|---|---|
| Ingredients | **% w/w** | **% w/w** |
| Cyclomethicone | 13.41 | 13.41 |
| Fragrance | 0.80 | 0.80 |
| C₁₂₋₁₅ alkyl benzoate | 2.00 | 2.00 |
| Dioctyl ether | 7.50 | 2.50 |
| Glycerol | 2.50 | 7.50 |
| ACH solution) | 20.00 | 20.00 |
| Water | 13.34 | 3.34 |
| Emulsifier | 0.25 | 0.25 |
| Silicone gum | 0.2 | 0.2 |
| Propellant | 50.00 | 50.00 |

**Table 8**

| Panel Score | Ex6 vs. C 9. |
|---|---|
| Wet on application | -1.3 |
| Wet 1 min | -1.0 |
| Wet 2 min | -0.4 |
| Sticky 1 min | -0.8 |
| Sticky 2 min | -1.2 |
| Greasy / oily 1 min | +0.2 |
| Greasy / oily 2 min | +0.5 |

The assessment shows that Example 6 was clearly perceived as less wet and sticky. Its slightly higher greasy/oily feel was not perceived as a negative difference.

### Example 7 and Comparisons C10 and C11

In this Example and comparisons, the sensory attributes of an invention composition were compared in the method described for Example 1 and Comparison C1, with those of a composition omitting diocyl ether or replacing it with hydrogenated polydecene. The compositions were summarised in Table 9 below and the assessments summarised in Table 10.

**Table 9**

| | **Ex 7** | **C10** | **C11** |
|---|---|---|---|
| Ingredients | **% w/w** | **% w/w** | **% w/w** |
| Cyclomethicone | 13.41 | 13.41 | 13.41 |
| Fragrance | 0.80 | 0.80 | 0.80 |
| C₁₂₋₁₅ alkyl benzoate | 2.00 | 2.00 | 2.00 |
| Dioctyl ether | 6.67 | 0 | 0 |
| Hydrogenated Polydecene | | | 6,67 |
| Glycerol | 3.33 | 10 | 3.33 |
| ACH solution) | 20.00 | 20.00 | 20.00 |
| Water | 13.34 | 3.34 | 3.34 |
| Emulsifier | 0.25 | 0.25 | 0.25 |
| Silicone gum | 0.2 | 0.2 | 0.2 |
| Propellant | 50.00 | 50.00 | 50.00 |

**Table 10**

| Panel Score | Ex7 vs C 10 | Ex7 vs C 11 |
|---|---|---|
| Wet on application | -0.5 | -0.5 |
| Wet 1 min | -0.8 | -0.8 |
| Wet 2 min | -0.6 | -0.8 |
| Sticky 1 min | -0.1 | -0.7 |
| Sticky 2 min | -0.0 | -1.0 |
| Greasy / oily 1 min | -0.1 | +0.9 |
| Greasy / oily 2 min | 0.0 | +0.9 |

The assessment showed that the formulation of Example 7 was less wet than the formulation (C10) from which dioctly ether was absent. There was no perceived difference in sticky or greasy feel at the time when this was assessed, though increased stickiness of the comparison formulation was expected to occur later.

The assessment showed that the formulation of Example 7 was both less wet and less sticky than that of the comparison C11 containing hydrogenated polydecene instead of dioctyl ether. Its higher greasy/oily feel was considered not to be a negative attribute because it is considered to provide a more silky feel during dry down.

### Example 8

In this Example, the composition employed an alternative polyhydric humectant, polyethylene glycol of average molecular weight 400, and summarised ion Table 11. The sensory attributes of the formulation Ex8 were compared with those of a previous Example, Ex5 and the results summarised in Table 12.

**Table 11**

| | **Ex8** |
|---|---|
| **Ingredients** | **% w/w** |
| Cyclomethicone | 5.06 |
| Fragrance | 0.80 |
| Dioctyl ether | 4.0 |
| Sunflower Seed Oil | 3.25 |
| Glycerol | |
| PEG 400 | 3.34 |
| ACH solution | 18.00 |
| Water | |
| Emulsifier | 0.25 |
| Silicone gum | 0.2 |
| Propellant | 65.00 |

**Table 12**

| Panel Score | Ex8 vs Ex5 |
|---|---|
| Wet on application | +0.3 |
| Wet 1 min | +0.3 |
| Wet 2 min | +0.1 |
| Sticky 1 min | -0.7 |
| Sticky 2 min | -0.1 |
| Greasy / oily 1 min | +0.2 |
| Greasy / oily 2 min | +0.1 |

The assessment shows that the invention formulation of Example 8 showed very similar characteristics to those of Example 5. After one minute, it was perceived to be even less sticky than that of the glycerol-containing invention composition.

## Claims

1. An antiperspirant aerosol composition comprising an antiperspirant base compositions and a liquefiable propellant **characterised in that** the proportion of the propellant in the aerosol composition is from 40 to 88% by weight and the base composition in the form of a liquid emulsion comprising a continuous oil phase, a dispersed aqueous phase and a silicone copolyol emulsifier, which base composition is suitable for combining with a propellant to form an aerosol composition, said aqueous phase representing from 45 to 80% of the base composition and comprising from 20 to 50% based on said aqueous phase of a water-soluble astringent antiperspirant active salt, and from 2 to 20% of a polyhydric humectant, based on the base composition said oil phase representing from 20 to 55% based on the base composition and comprising a volatile silicone oil in an proportion of at least 25% of the oil phase and a sensory modifying oil selected from dialkyl ethers and dialkyl carbonates having a boiling point of at least 280°C in an proportion of at least 20% of the oil phase and preferably in a weight ratio to the polyhydric humectant of from 3:4 to 4:1,
and said emulsifier being present in an amount of at least 0.15% of the base composition
all %s being by weight.

2. A composition according to claim 1 in which the oil phase represents from 30 to 50% and preferably from 32 to 40% by weight of the base composition

3. A composition according to either preceding claim in which the volatile silicone oil is present at a concentration of from 30 to 70% by weight of the oil phase.

4. A composition according to any preceding claim in which the sensory modifying oil has a boiling point of from 280°C to 420°C.

5. A composition according to claim 4 in which the sensory modifying oil comprises at least half by weight of dioctyl ether and/or dioctyl carbonate.

6. A composition according to any preceding claim in which the sensory modifying oil is present at a concentration of from 20 to 35% by weight of the oil phase.

7. A composition according to any preceding claim in which polyhydric humectant is present in an amount of from 3 to 15% by weight of the base composition.

8. A composition according to any preceding claim in which the antiperspirant active is present in an amount of from 15 to 40% by weight of the aqueous phase.

9. A composition according to any preceding claim in which the antiperspirant active is present at concentration of from 30 to 45% by weight of the aqueous phase.

10. A composition according to any preceding claim in which the polyhydric humectant is present in a weight ratio to the antiperspirant active of from 0.3:1 to 0.75:1.

11. A composition according to any preceding claim in which the polyhydric humectant comprises glycerol.

12. A composition according to any of claims 1 to 10 in which the polyhydric humectant comprises polyethylene glycol.

13. A composition according to claim 12 in which the polyethylene glycol contain on average from 4 to 12 glycol mers.

14. A composition according to any preceding claim in which the antiperspirant active is an aluminium chlorohydrate.

15. A composition according to any preceding claim in which the sensory modifying oil is present in a weight ratio to the polyhydric humectant of from 6:5 to 2:1.

16. A composition according to any preceding claim in which the oil phase additionally comprises at least one ester oil selected from triglyceride oils, and ester oils having a refractive index of at least 1.48.

17. A composition according to claim 16 in which the ester oil is present in a concentration of from 5 to 35% by weight of the oil phase.

18. A composition according to any preceding claim further containing a spray modifier comprising a silicone gum at a concentration of up to 1.35% by weight of the oil phase.

19. A. composition according to claim 18 in which the silicone copolyol is a dimethicone copolyol or an alkylmethicone copolyol.

20. A composition according to claim 18 or 19 in which the emulsifier is present at a concentration of from 0.3 to 1.0% by weight of the base composition.

21. A composition according to any preceding claim containing up to 1/3rd by weight of said base composition in which the oil and aqueous phases are present in a weight ratio of from 1:2 to 3:4.

22. A composition according to claim 21 in which the concentration of volatile silicone in the oil phase is from 30 to 45%.

23. An aerosol composition according to any of claims 1 to 20 containing from less than 1/3rd to 9/20ths by weight of said base composition in which the oil and aqueous phases are present in a weight ratio of from 3:5 to 1:1.

24. A composition according to claim 23 in which the concentration of volatile silicone in the oil phase is from 40 to 50%.

25. An aerosol composition according to any of claims 1 to 20 containing from more than 9/20ths to 3/5ths by weight of said base composition in which the oil and aqueous phases are present in a weight ratio of from 1:2 to 1:1.

26. A composition according to any preceding claim in which the concentration of sensory modifying oil in the oil phase is from 22 to 33%.

27. An aerosol composition according to claim 22 containing from 3 to 18% by weight of the antiperspirant active.

28. A composition according to claim 27 containing from 7.5 to 18% by weight of aluminium chlorohydrate.

29. A non-therapeutic method of at least inhibiting perspiration or body malodour formation comprising topically applying to human skin a composition according to any preceding claim.

## Patentansprüche

1. Schweißhemmende Aerosol-Zusammensetzung, umfassend eine schweißhemmende Basiszusammensetzung und ein verflüssigbares Treibmittel, **dadurch gekennzeichnet, dass** der Anteil des Treibmittels in der Aerosol-Zusammensetzung 40 bis 88 Gew.-% ist und die Basiszusammensetzung in der Form einer flüssigen Emulsion ist, die eine kontinuierliche Ölphase, eine dispergierte wässrige Phase und einen Silikoncopolyol-Emulgator umfasst, wobei die Basiszusammensetzung zum Kombinieren mit einem Treibmittel unter Bildung einer Aerosol-Zusammensetzung geeignet ist, wobei die wässrige Phase 45 bis 80 % der Basiszusammensetzung darstellt und 20 bis 50 %, bezogen auf die wässrige. Phase, eines wasserlöslichen adstringierenden schweißhemmenden aktiven Salzes und 2 bis 20 % eines mehrere HydroxylGruppen enthaltenden Feuchthaltemittels, bezogen auf die Basiszusammensetzung, umfasst, wobei die Ölphase 20 bis 55 %, bezogen auf die Basiszusammensetzung, darstellt und ein flüchtiges Silikonöl in einem Anteil von wenigstens 25 % der Ölphase und ein sensorisch modifizierendes Öl, ausgewählt aus Dialkylethern und Dialkylcarbonaten mit einem Siedepunkt von wenigstens 280 °C in einem Anteil von wenigstens 20 % der Ölphase und vorzugsweise in einem Gewichtsverhältnis zu dem mehrere Hydroxylgruppen enthaltenden Feuchthaltemittel von 3:4 bis 4:1 umfasst,
und wobei der Emulgator in einer Menge von wenigstens 0,15 % der Basiszusammensetzung vorliegt, wobei alle % Gewichtsprozente sind.

2. Zusammensetzung gemäß Anspruch 1, wobei die Ölphase 30 bis 50 Gew.-% und vorzugsweise 32 bis 40 Gew.-% der Basiszusammensetzung darstellt.

3. Zusammensetzung gemäß einem vorangehenden Anspruch, wobei das flüchtige Silikonöl in einer Konzentration von 30 bis 70 Gew.-% der Ölphase vorliegt.

4. Zusammensetzung gemäß einem vorangehenden Anspruch, wobei das sensorisch modifizierende Öl einen Siedepunkt von 280 °C bis 420 °C hat.

5. Zusammensetzung gemäß Anspruch 4, wobei das sensorisch modifizierende Öl wenigstens das halbe Gewicht von Dioctylether und/oder Dioctylcarbonat umfasst.

6. Zusammensetzung gemäß einem vorangehenden Anspruch, wobei das sensorisch modifizierende Öl in einer Konzentration von 20 bis 35 Gew.-% der Ölphase vorliegt.

7. Zusammensetzung gemäß einem vorangehenden Anspruch, wobei das mehrere Hydroxylgruppen enthaltende Feuchthaltemittel in einer Menge von 3 bis 15 Gew.-% der Basiszusammensetzung vorliegt.

8. Zusammensetzung gemäß einem vorangehenden Anspruch, wobei der schweißhemmende Wirkstoff in einer Menge von 15 bis 40 Gew.-% der wässrigen Phase vorliegt.

9. Zusammensetzung gemäß einem vorangehenden Anspruch, wobei der schweißhemmende Wirkstoff in einer Konzentration von 30 bis 45 Gew.-% der wässrigen Phase vorliegt.

10. Zusammensetzung gemäß einem vorangehenden Anspruch, wobei das mehrere Hydroxylgruppen enthaltende Feuchthaltemittel in einem Gewichtsverhältnis zu dem schweißhemmenden Wirkstoff von 0,3:1 bis 0,75:1 vorliegt.

11. Zusammensetzung gemäß einem vorangehenden Anspruch, wobei das mehrere Hydroxylgruppen enthaltende Feuchthaltemittel Glycerin umfasst.

12. Zusammensetzung gemäß einem der Ansprüche 1 bis 10, wobei das mehrere Hydroxylgruppen enthaltende Feuchthaltemittel Polyethylenglykol umfasst.

13. Zusammensetzung gemäß Anspruch 12, wobei das Polyethylenglykol durchschnittlich 4 bis 12 Glykolmere enthält.

14. Zusammensetzung gemäß einem vorangehenden Anspruch, wobei der schweißhemmende Wirkstoff Aluminiumchlorhydrat ist.

15. Zusammensetzung gemäß einem vorangehenden Anspruch, wobei das sensorisch modifizierende Öl in einem Gewichtsverhältnis zu dem mehrere Hydroxylgruppen enthaltenden Feuchthaltemittel von 6:5 bis 2:1 vorliegt.

16. Zusammensetzung gemäß einem vorangehenden Anspruch, wobei die Ölphase zusätzlich wenigstens ein Esteröl, ausgewählt aus Triglyceridölen, und Esterölen mit einem Brechnungsindex von wenigstens 1,48 umfasst.

17. Zusammensetzung gemäß Anspruch 16, wobei das Esteröl in einer Konzentration von 5 bis 35 Gew.-% der Ölphase vorliegt.

18. Zusammensetzung gemäß einem vorangehenden Anspruch, die außerdem ein Sprühmodifizierungsmittel, das einen Silikongummi umfasst, in einer Konzentration von bis zu 1,35 Gew.-% der Ölphase enthält.

19. Zusammensetzung gemäß Anspruch 18, wobei das Silikoncopolyol ein Dimethiconcopolyol oder ein Alkylmethiconcopolyol ist.

20. Zusammensetzung gemäß Anspruch 18 oder 19, wobei der Emulgator in einer Konzentration von 0,3 bis 1,0 Gew.-% der Basiszusammensetzung vorliegt.

21. Zusammensetzung gemäß einem vorangehenden Anspruch, die bis zu 1/3, bezogen auf das Gewicht, der Basiszusammensetzung enthält, wobei die Öl- und wässrige Phase in einem Gewichtsverhältnis von 1:2 bis 3:4 vorliegen.

22. Zusammensetzung gemäß Anspruch 21, wobei die Konzentration an flüchtigem Silikon in der Ölphase 30 bis 45 % beträgt.

23. Aerosol-Zusammensetzung gemäß einem der Anspruche 1 bis 20, die weniger als 1/3 bis 9/20, bezogen auf das Gewicht, der Basiszusammensetzung enthält, in der die Öl- und wässrige Phase in einem Gewichtsverhältnis von 3:5 bis 1:1 vorliegen.

24. Zusammensetzung gemäß Anspruch 23, wobei die Konzentration an flüchtigem Silikon in der Ölphase 40 bis 50 % beträgt.

25. Aerosol-Zusammensetzung gemäß einem der Ansprüche 1 bis 20, die mehr als 9/20 bis 3/5, bezogen auf das Gewicht, der Basiszusammensetzung enthält, wobei die Öl- und wässrige Phase in einem Gewichtsverhältnis von 1:2 bis 1:1 vorliegen.

26. Zusammensetzung gemäß einem vorangehenden Anspruch, wobei die Konzentration an sensorisch modifizierendem Öl in der Ölphase 22 bis 33 % beträgt.

27. Aerosol-Zusammensetzung gemäß Anspruch 22, die 3 bis 18 Gew.-% des schweißhemmenden Wirkstoffs enthält.

28. Zusammensetzung gemäß Anspruch 27, die 7,5 bis 18 Gew.-% Aluminiumchlorhydrat enthält.

29. Nicht-therapeutisches Verfahren zum wenigstens Inhibieren der Perspiration oder der Körpergeruchsbildung, das topisches Auftragen einer Zusammensetzung gemäß einem vorangehenden Anspruch auf menschliche Haut umfasst.

## Revendications

1. Composition aérosol antiperspirante comprenant une composition de base antiperspirante et un gaz propulseur liquéfiable, **caractérisée en ce que** la proportion du gaz propulseur dans la composition aérosol est de 40 à 88 % en poids et **en ce que** la composition de base est sous la forme d'une émulsion liquide comprenant une phase huileuse continue, une phase aqueuse dispersée et un émulsifiant de type silicone copolyol,
ladite composition de base se prêtant à une combinaison avec un gaz propulseur pour former une composition aérosol,
ladite phase aqueuse représentant de 45 à à 80 % de la composition de base et comprenant de 20 à 50 %, sur la base de ladite phase aqueuse, d'un principe actif antiperspirant sous la forme d'un sel astringent soluble dans l'eau, et de 2 à 20 % d'un agent hydratant polyhydrique, sur la base de la composition de base,
ladite phase huileuse représentant de 20 à 55 %, sur la base de la composition de base, et comprenant une huile silicone volatile en une proportion d'au moins 25 % de la phase huileuse et une huile modifiant l'impression sensorielle choisie parmi les éthers de dialkyle et les carbonates de dialkyle ayant un point d'ébullition d'au moins 280°C en une proportion d'au moins 20 % de la phase huileuse et, de préférence, dans un rapport en poids à l'agent hydratant polyhydrique de 3:4 à 4;1, et ledit émulsifiant étant présent en une quantité d'au moins 0,15 % de la composition de base tous les % étant en poids.

2. Composition selon la revendication 1, dans laquelle la phase huileuse représente de 30 à 50 %, et de préférence, de 32 à 40 % en poids de la composition de base.

3. Composition selon l'une quelconque des revendications précédentes, dans laquelle l'huile silicone volatile est présente à une concentration de 30 à 70 % en poids de la phase huileuse.

4. Composition selon l'une quelconque des revendications précédentes, dans laquelle l'huile modifiant l'impression sensorielle a un point d'ébullition de 280 à 420 °C.

5. Composition selon la revendication 4, dans laquelle l'huile modifiant l'impression sensorielle comprend au moins la moitié en poids d'éther de dioctyle et/ou de carbonate de dioctyle.

6. Composition selon l'une quelconque des revendications précédentes, dans laquelle l'huile modifiant l'impression sensorielle est présente à une concentration de 20 à 35 % en poids de la phase huileuse.

7. Composition selon l'une quelconque des revendications précédentes, dans laquelle l'agent hydratant polyhydrique est présent en une quantité de 3 à 15 % en poids de la composition de base.

8. Composition selon l'une quelconque des revendications précédentes, dans laquelle le principe actif antiperspirant est présent en une quantité de 15 à 40 % en poids de la phase aqueuse.

9. Composition selon l'une quelconque des revendications précédentes, dans laquelle le principe actif antiperspirant est présent à une concentration de 30 à 45 % en poids de la phase aqueuse.

10. Composition selon l'une quelconque des revendications précédentes, dans laquelle l'agent hydratant polyhydrique est présent dans un rapport en poids au principe actif antiperspirant de 0,3:1 à 0,75:1.

11. Composition selon l'une quelconque des revendications précédentes, dans laquelle l'agent hydratant polyhydrique comprend le glycérol.

12. Composition selon l'une quelconque des revendications 1 à 10, dans laquelle l'agent hydratant polyhydrique comprend le polyéthylène glycol.

13. Composition selon la revendication 12, dans laquelle le polyéthylène glycol contient en moyenne de 4 à 12 motifs monomériques de glycol.

14. Composition selon l'une quelconque des revendications précédentes, dans laquelle le principe actif antiperspirant est un chlorhydrate d'aluminium.

15. Composition selon l'une quelconque des revendications précédentes, dans laquelle l'huile modifiant l'impression sensorielle est présente dans un rapport en poids à l'agent hydratant polyhydrique de 6:5 à 2:1.

16. Composition selon l'une quelconque des revendications précédentes, dans laquelle la phase huileuse comprend, en plus, au moins une huile de type ester choisie parmi les huiles tri-glycéridiques, et les huiles de type ester ayant un indice de réfraction d'au moins 1,48.

17. Composition selon la revendication 16, dans laquelle l'huile de type ester est présente à une concentration de 5 à 35 % en poids de la phase huileuse.

18. Composition selon l'une quelconque des revendications précédentes contenant, en outre, un modificateur de pulvérisation comprenant une gomme silicone à une concentration pouvant aller jusqu'à 1,35 % en poids de la phase huileuse.

19. Composition selon la revendication 18, dans laquelle le silicone copolyol est un diméthicone copolyol ou un alkylméthicone copolyol.

20. Composition selon la revendication 18 ou 19, dans laquelle l'émulsifiant est présent à une concentration de 0,3 à 1,0 % en poids de la composition de base.

21. Composition selon l'une quelconque des revendications précédentes contenant jusqu'à 1/3 en poids de ladite composition de base dans laquelle les phases huileuse et aqueuse sont présentes dans un rapport en poids de 1:2 à 3:4.

22. Composition selon la revendication 21, dans laquelle la concentration de la silicone volatile dans la phase huileuse est de 30 à 45 %.

23. Composition aérosol selon l'une quelconque des revendications 1 à 20 contenant de moins d'1/3 à 9/20^{ème} en poids de ladite composition de base dans laquelle les phases huileuse et aqueuse sont présentes dans un rapport en poids de 3:5 à 1:1.

24. Composition selon la revendication 23, dans laquelle la concentration de la silicone volatile dans la phase huileuse est de 40 à 50 %.

25. Composition aérosol selon l'une quelconque des revendications 1 à 20 contenant de plus de 9/20^{ème} à 3/5^{ème} en poids de ladite composition de base dans laquelle les phases huileuse et aqueuse sont présentes dans un rapport en poids de 1:2 à 1:1.

26. Composition selon l'une quelconque des revendications précédentes, dans laquelle la concentration de l'huile modifiant l'impression sensorielle dans la phase huileuse est de 22 à 33 %.

27. Composition aérosol selon la revendication 22 contenant de 3 à 18 % en poids de principe actif antiperspirant.

28. Composition selon la revendication 27, contenant de 7,5 à 18 % en poids de chlorhydrate d'aluminium.

29. Procédé non thérapeutique permettant au moins d'inhiber la formation de transpiration ou de mauvaises odeurs corporelles comprenant l'application topique à la peau humaine d'une composition selon l'une quelconque des revendications précédentes.
